# EUROPÄISCHE PATENTSCHRIFT

(11) **EP 2 384 723 B1**
(45) Veröffentlichungstag und Bekanntmachung des Hinweises auf die Patenterteilung: **12.12.2012**
(21) Anmeldenummer: 10162347.8
(22) Anmeldetag: 07.05.2010
(51) Int. Cl.: A61F 2/34, A61F 2/46

(54) **Verfahren zur Herstellung eines Implantats sowie Implantat mit mindestens einer Sollbruchlinie**
Method for producing an implant and implant with at least one predetermined breaking line
Procédé de fabrication d'un implant et implant doté d'au moins une ligne de rupture prévue

(43) Veröffentlichungstag der Anmeldung: 09.11.2011
(73) Patentinhaber: Jossi Holding AG, 8546 Islikon (CH)
(72) Erfinder: Gugler, Christian, 8500 Frauenfeld (CH); Nadler, Daniel, 8442 Hettlingen (CH); Meyenhofer, Andreas, 8255 Schlattingen (CH); Bodmer, Simon, 8400 Winterthur (CH)
(74) Vertreter: Hepp Wenger Ryffel AG

(56) Entgegenhaltungen:
- EP-A1- 1 728 489
- WO-A1-2010/023447
- DE-A1-102008 022 329
- US-A- 5 370 702

## Beschreibung

Die vorliegende Erfindung betrifft ein Herstellungsverfahren für ein Implantat gemäss dem Oberbegriff von Anspruch 1.

Bei Implantaten kann es von Vorteil sein, wenn z.B. einzelne Schraubenlöcher oder Öffnungen selektiv geöffnet werden können. Dies ist besonders bei Hüftgelenkpfannen wichtig, da durch unbenutzte Schraubenlöcher Abriebpartikel aus der Gelenkpfanne in den Bereich zwischen Implantat und Knochen gelangen können. Aber auch bei Osteosyntheseplatten kann es von Vorteil sein, selektiv einzelne Schraubenöffnungen verschliessen oder bestimmte Bereiche des Implantats entfernen zu können.

In der FR2826865 wird deshalb vorgeschlagen, Hüftgelenkpfannen herzustellen, deren Schraubenlöcher durch Abdeckungen verschlossen sind, die durch einzelne Schweisspunkte an der Aussenseite der Gelenkpfanne befestigt sind. Wird eines der Schraubenlöcher gebraucht, kann die Abdeckung durch Brechen dieser Schweisspunkte entfernt werden.

Die US 5,782,929 offenbart eine Hüftgelenkpfanne aus Titan, bei der die Schraubenöffnungen durch Titanstopfen, die mit der Gelenkpfanne versintert wurden, verschlossen sind. Die Stopfen werden dabei separat von der Hüftgelenkpfanne gefertigt und erst nachträglich in die Öffnungen eingesetzt. Durch mehrstündiges Versintern in einem Ofen bei ca. 1200°C werden die Stopfen mit der Pfanne verbunden.

Nachteilig an den beiden beschriebenen Verfahren ist, dass die Herstellung solcher Gelenkpfannen mehrere Schritte umfasst und demnach entsprechend aufwändig ist. Ausserdem kann die Stärke der Verbindung der Abdeckungen bez. Stopfen zur Gelenkpfanne je nach Öffnung unterschiedlich sein.

In der WO 2010/023447 wird eine Prothese beschrieben, die aus mit Carbonfasern verstärktem Polyetheretherketon (CFR-PEEK) besteht. Die Schraubenöffnungen können bei einer Ausführungsform durch ausbrechbare Stücke verschlossen sein. Dabei wird bei der Herstellung im Bereich der Öffnungen durch gezielte Reduktion der Materialstärke eine Sollbruchstelle geschaffen. Die Prothese kann dabei aus einem Stück spanabhebend oder auch durch Spritzguss hergestellt werden.

Von der EP 0 701 420 ist bekannt, dass Hüftgelenkpfannen mit herausbrechbaren Abdeckungen durch Bohren von zylindrischen Sacklöchern auf der Innenseite der Pfanne hergestellt werden können. Dabei werden die Sacklöcher so angebracht, dass zwischen der Aussenfläche der Gelenkpfanne und dem Ende des Lochs eine Materialstärke von unter 0.65mm verbleibt. Dadurch lässt sich die Abdeckung des Sacklochs bei Bedarf herausbrechen.

Die FR 2 838 329 beschreibt eine Hüftgelenkpfanne mit Schraubenöffnungen, die durch Abdeckungen verschlossen sind, die mit der Hüftgelenkpfanne materiell in Verbindung stehen. Die Abdeckungen sind dabei durch einen Bereich mit verminderter Materialstärke begrenzt, was ein einfaches Herausbrechen der Abdeckungen ermöglicht. Die Abdeckungen weisen gegen das Innere der Gelenkpfanne zusätzliche Vorsprünge auf, in die weitere Stopfen eingesetzt werden können.

Ferner ist ebenfalls aus der US 5,370,702 bekannt, dass entfernbare Abdeckungen von Schraubenöffnungen durch verminderte Materialstärke hergestellt werden können. Die in diesem Patent offenbarten Abdeckungen weisen in Richtung des Innern einer Hüftgelenkpfanne dornartige Fortsätze auf, die durch ein geeignetes Werkzeug ergriffen werden können und das Herausbrechen der Abdeckungen erleichtern.

Die EP 1 338 256 beschreibt eine Hüftgelenkpfanne mit ausstanzbaren Schraubenöffnungen. Die Materialstärke der Hüftgelenkpfanne ist dabei im Bereich dieser Öffnungen reduziert, um deren Ausstanzen zu erleichtern. Ferner beschreibt dieses Dokument Werkzeuge, um die Schraubenöffnungen auszustanzen. Der Bereich mit reduzierter Materialstärke kann dabei zusätzlich über eine kreisförmige Kerbe verfügen, um die zur Entfernung nötige Ausstanzkraft zusätzlich zu reduzieren.

Die genannten Abdeckungen haben den wesentlichen Nachteil, dass deren Herstellung durch spanabhebende Verfahren und den erforderlichen geringen Materialstärken Probleme bereitet, da diese Materialstärken bei einer Mehrfach-Aufspannung oft innerhalb der Fehlertoleranz von gebräuchlichen Maschinen liegen. Ausserdem ist wegen der geforderten Präzision deren Herstellung aufwändig und teuer.

Eine Aufgabe der vorliegenden Erfindung liegt nun darin, ein Verfahren der eingangs genannten Art zu schaffen, das die genannten Nachteile vermeidet und das eine sehr präzise Dimensionierung der Sollbruchlinie ermöglicht, ohne dass benachbarte Bereiche deformiert, bez. in ihrer Masshaltigkeit beeinfluss werden oder anderweitig, z.B. thermisch überbeansprucht werden. Ausserdem soll das Verfahren kostengünstig sein und sich für Implantate unterschiedlicher Konfiguration eignen. Diese Aufgabe wird mit einem Verfahren gemäss Anspruch 1 gelöst.

Beim erfindungsgemässen Verfahren wird in einem ersten, vorzugsweise spanabhebenden Fertigungsverfahren ein Halbfabrikat des Implantats hergestellt. Anschliessend wird durch Materialumformung eine Sollbruchlinie durch eine Reduzierung der Materialstärke erzeugt.

Der erste Verfahrensschritt kann durch ein bekanntes Fertigungsverfahren, wie beispielsweise Fräsen und/oder Drehen erfolgen. Anschliessend wird dann die Sollbruchlinie hergestellt. Je nach Ausgestaltung des Implantats kann es vorteilhaft sein, wenn die zum Anbringen der Sollbruchlinie vorgesehenen Wandabschnitte durch das erste Fertigungsverfahren weitgehend vorgeformt wurden, beispielsweise durch Anbringen einer Vertiefung oder ähnliches. Besonders bevorzugt wird im Halbfabrikat zunächst mindestens ein Sackloch erzeugt, an dessen Grund im zweiten Verfahrensschritt eine Sollbruchlinie eingeprägt wird.

Besonders vorteilhaft beim erfindungsgemässen Verfahren ist der Umstand, dass durch Materialumformung bei der Sollbruchlinie rationell auch sehr geringe Wandstärken erreicht werden können. Ausserdem kann eine Sollbruchlinie auch auf ein Implantat mit komplexer Oberflächengeometrie aufgebracht werden. Durch den geringen Verschleiss der benutzten Prägestempel lässt sich zudem eine sehr hohe Reproduzierbarkeit erreichen.

Bevorzugt erfolgt beim erfindungsgemässen Verfahren die Materialumformung durch Druckformung im kalten wie auch im warmen Zustand. Dabei können je nach zu erzielender Geometrie unterschiedliche Prägestempel verwendet werden. Durch Variation des Prägedrucks und/oder des Verfahrwegs lässt sich zudem die Wandstärke im Bereich der Sollbruchlinie variieren. Bei hochfesten Materialien kann durch Warmumformung die Prägekraft reduziert werden oder der Umformungsgrad gezielt erhöht werden.

Im erfindungsgemässen Verfahren erfolgt die Materialumformung bevorzugterweise derart, dass die Umformkräfte beziehungsweise der Materialfluss in den Verschlussabschnitt geleitet werden. Der Prägestempel kann dabei derart beschaffen sein, dass die durch den Prägedruck entstehenden Prägekräfte und der daraus folgende Materialfluss in den Verschlussabschnitt geleitet werden können. Dadurch wird verhindert, dass sich störende Verformungen z.B. am Implantatkörper bilden. Ausserdem lassen sich dadurch sehr dünne und beliebig verlaufende, hochpräzis abbrechende Sollbruchlinien herstellen.

Der entfernbare Verschlussabschnitt wird bevorzugter Weise derart hergestellt, dass wenigstens ein Angriffsmittel zum Ansetzen eines Instruments gebildet wird. Dieses Angriffsmittel weist vorzugsweise die Form eines Materialvorsprungs mit Hinterschnitt auf. Das Angriffsmittel kann jedoch auch andere Formen aufweisen, wie beispielsweise Schlitze zum Eingreifen eines Schraubenziehers, Gewinde oder ähnliches. Ein solches Angriffsmittel hat den Vorteil, dass dadurch das Herausbrechen des Verschlussabschnitts erleichtert wird. Ferner lässt sich durch ein zur Sollbruchlinie asymetrisch angeordnetes Angriffsmittel die zum Entfernen des Verschlussabschnitts maximal nötige Kraft um bis zu 80% reduzieren.

Die Wandstärke im Bereich einer Sollbruchlinie wird durch die Materialumformung bevorzugt auf 0.01mm bis 1mm, besonders bevorzugt auf 0.1mm bis 0.3mm reduziert. Diese Wandstärke reicht einerseits aus, um ein unbeabsichtigtes Ausbrechen des Verschlussabschnitts zu verhindern, ist andererseits jedoch dünn genug, um das Freilegen einer Öffnung oder Aussparung unter Krafteinwirkung ohne maschineller Hilfe zu ermöglichen. Dies erlaubt es z.B. einzelne Verschlussabschnitte während der Operation, unter Umständen sogar bei bereits eingesetztem Implantat zu entfernen.

Ferner kann das Verfahren bevorzugt derart ausgeführt werden, dass der Verschlussabschnitt durch die Materialumformung zusätzlich konvex durchgebogen wird. Durch die Umformung können alternativ auch konkav durchgebogene oder gewellte Verschlussabschnitte erzeugt werden. Eine konkave oder konvexe Durchbiegung des Verschlussabschnitts kann deren Zugänglichkeit zum Entfernen sowie das Entfernen selber vereinfachen.

Bei der Materialumformung kann der die Sollbruchlinie umgebende Wandabschnitt abgestützt werden. Dies verhindert, dass sich das umliegende Material durch den Pressdruck verformt.

Eine weitere Aufgabe der vorliegenden Erfindung besteht darin, ein Implantat zu schaffen, welches einen Wandabschnitt mit mindestens einer Sollbruchlinie aufweist. Diese Aufgabe wird durch ein Implantat gemäss Anspruch 8 gelöst.

Das erfindungsgemässe Implantat weist dabei einen Wandabschnitt mit mindestens einer, mittels Schwächung der Materialstärke durch Materialumformung geschaffenen Sollbruchlinie auf.

Bevorzugt ist das Implantat dabei eine Gelenkpfanne, besonders bevorzugt eine Hüftgelenkpfanne. Das Implantat kann alternativ aber auch als Schultergelenkschale, Femur- oder Humerusschaftimplantat, Knieprothese, Marknagel oder Osteosyntheseplatte ausgebildet sein.

Der durch die Sollbruchlinie abgegrenzte Verschlussabschnitt ist vorzugsweise kreisförmig, oval, elliptisch, polygonal oder als Kombination davon ausgebildet.

Der Verschlussabschnitt kann alternativ auch derart auf dem Implantat aufgebracht sein, dass dieser mindestens teilweise durch eine Aussenkante des Implantats begrenzt ist. Dadurch lassen sich entfernbare Randbereiche an einem Implantat herstellen, beispielsweise an einer Osteonsyntheseplatte.

Das Implantat besteht vorzugsweise aus einem metallischen Werkstoff, besonders bevorzugt aus Titan, einer Titanlegierung, einer Cobalt-Chromlegierung, einer Magnesium- oder Stahllegierung. Alternativ kann das Implantat auch aus einem nichtmetallischen Werkstoff bestehen, wie z.B. Polyetheretherketon (PEEK) oder Polyoxymethylen (POM).

Bevorzugt weist der Verschlussabschnitt Angriffsmittel zum Aufsetzen eines Instruments auf. Besonders bevorzugt ist dieses Angriffsmittel als Materialvorsprung mit Hinterschnitt ausgebildet. Dies ermöglicht ein einfaches und sicheres Entfernen des Verschlussabschnitts durch ein Instrument.

Eine Vorrichtung zu Herstellung eines erfindungsgemässen Implantats weist zwei als Matrize und Patrize oder zwei als Patrize ausgebildete Prägstempel auf. Zusätzlich kann die Vorrichtung ein Stützelement aufweisen, welches den der Sollbruchstelle benachbarten Wandabschnitt abstützt. Dies verhindert die Bildung von ungewollten Verformungen ausserhalb der Sollbruchlinie und erlaubt ein exaktes Positionieren des Implantathalbfabrikats in der Vorrichtung.

Ein Instrument zum Freilegen einer Öffnung verfügt über einen länglichen Griff sowie über Eingriffsmittel. Die Eingriffmittel sind wieder lösbar mit dem Verschlussabschnitt verbindbar.

Über den länglichen Griff kann eine Kraft ausgeübt werden, um den Verschlussabschnitt aus der Wand des Implantats zu entfernen. Die Eingriffmittel dienen dabei einerseits zum Übertragen der Kraft vom Griff auf den Verschlussabschnitt und andererseits kann der Verschlussabschnitt damit sicher und einfach aus der Implantatwand entfernt werden.

Bevorzugt sind die Eingriffmittel derart ausgebildet, dass diese mit einem auf dem Verschlussabschnitt angeordneten Angriffmittel verbindbar sind. Dadurch kann der Verschlussabschnitt unverlierbar mit dem Instrument verbunden werden, was auch eine Entfernung eines Verschlussabschnitts bei bereits eingesetztem Implantat ermöglicht.

Ein erfindungsgemässer Verschlussabschnitt kann auch durch beaufschlagen mit Druck- oder Zugkräften entfernt werden. Dies kann beispielsweise durch Drücken oder Schlagen sowie durch Ziehen erfolgen. Alternativ kann ein erfindungsgemässer Verschlussabschnitt auch durch Biege- oder Drehkräfte entfernt werden.

Weitere Vorteile und Einzelmerkmale der Erfindung ergeben sich aus der nachfolgenden Beschreibung von Ausführungsbeispielen und aus den Zeichnungen. Es zeigen:
- Fig. 1:: einen schematischen Querschnitt durch ein erfindungsgemässes Implantat,
- Fig. 2:: einen Querschnitt durch einen Verschlussabschnitt mit Sollbruchlinie in stark vergrösserter Darstellung,
- Fig. 3a-3e:: fünf verschiedene schematische Querschnitte durch entfernbare Verschlussabschnitte mit Sollbruchlinien,
- Fig. 4:: eine schematische Darstellung einer Vorrichtung zur Herstellung eines Implantats,
- Fig. 5a/5b:: schematische Darstellungen des Umformprozesses des erfindungsgemässen Verfahrens, und
- Fig. 6:: einen Teilquerschnitt durch ein Implantat mit einem angesetzten Instrument zum Ausbrechen eines erfindungsgemässen Verschlussabschnitts.

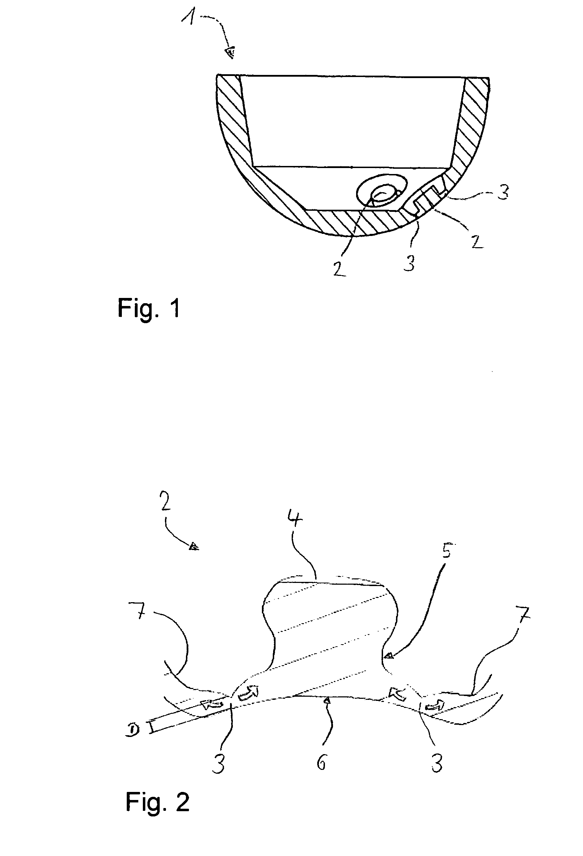

Figur 1 stellt einen schematischen Querschnitt eines Implantats 1 dar, bei welchem durch das erfindungsgemässe Verfahren durch Sollbruchlinien 3 abgegrenzte Verschlussabschnitte 2 angebracht sind. Exemplarisch ist das Implantat 1 als Hüftgelenkpfanne ausgebildet. Alternativ kann es sich beim Implantat jedoch auch um ein anderes Implantat handeln, wie beispielsweise eine Osteosyntheseplatte. Beispielhaft weist das Implantat 1 zwei Verschlussabschnitte 2 auf. Ein Implantat 1 kann jedoch auch nur einen Verschlussabschnitt 2 oder mehr als zwei Verschlussabschnitte 2, aufweisen. Die Verschlussabschnitte 2 können dabei in beliebiger Anordnung auf der Oberfläche des Implantats 1 angeordnet werden.

Figur 2 zeigt einen schematischen Schnitt durch einen Verschlussabschnitt eines Implantats, bei welchem mit dem erfindungsgemässen Verfahren ein durch eine Sollbruchlinie 3 abgegrenzter Verschlussabschnitt 2 angebracht wurde. Die Sollbruchlinie 3 weist gegenüber dem umgebenden Wandabschnitt 7 eine geringere Materialstärke D auf. Typischerweise ist die Materialstärke D dabei kleiner als 1mm. Der durch das Umformverfahren erfolgte Materialfluss, exemplarisch durch Pfeile dargestellt, wird dabei bevorzugt gezielt in den Verschlussabschnitt 2 geleitet. Alternativ kann der Materialfluss aber auch gezielt in den umgebenden Wandabschnitt geleitet werden. Der Verschlussabschnitt 2 weist ein Angriffsmittel 4 auf, welches in diesem Beispiel als Materialvorsprung mit Hinterschnitt 5 ausgebildet ist. Das Angriffsmittel 4 kann alternativ auch andere Formen aufweisen. Der Verschlussabschnitt 2 weist dabei zusätzlich eine konkave Wölbung 6 auf. Die Wölbung 6 kann alternativ auch konvex oder gewellt ausgestaltet sein. Der Verschlussabschnitt 2 kann alternativ auch flach ausgestaltet sein.

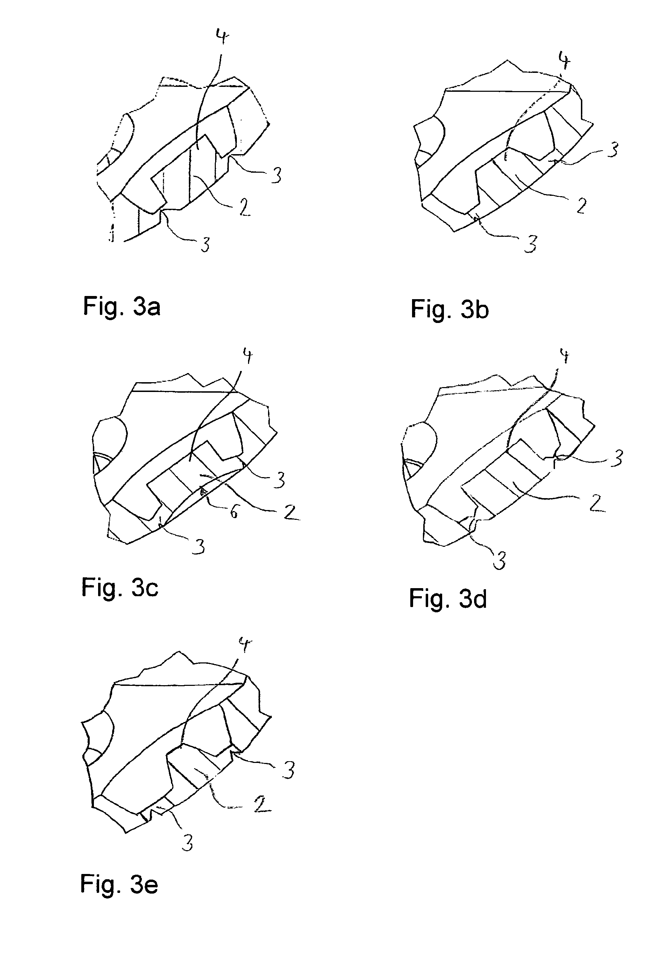

Die Figuren 3a bis 3e zeigen verschiedene Ausführungsformen von Verschlussabschnitten 2 eines erfindungsgemässen Implantats. Die Ausführungsform der Figur 3a weist dabei ein Angriffsmittel 4 auf, welches zylindrisch oder prismatisch ausgebildet ist. Figur 3b zeigt eine Ausführungsform mit kegelstumpfförmigem Angriffsmittel 4. Die Sollbruchlinie 3, die den Verschlussabschnitt 2 abgrenzt weist bei dieser Ausführungsform eine grössere Materialstärke auf, als die Ausführungsform der Figur 3a. Eine weitere Variante zeigt Figur 3c. Bei dieser Variante weist die Aussenseite des Verschlussabschnitts 4 eine konvexe Wölbung 6 auf. Der in Figur 3a gezeigte Verschlussabschnitt 2 kann dabei einen Zwischenschritt bei der Herstellung des Verschlussabschnitts 2 der Figur 3c sein. Figur 3d zeigt eine weitere Ausführungsform eines Verschlussabschnitts 2 bei dem die Materialstärke der Sollbruchlinie 3 auf beiden Seiten der Wand des Implantats reduziert wurde. Figur 3e zeigt eine weitere Ausführungsform eines Verschlussabschnitts 3. Bei dieser Ausführungsform ist das Angriffsmittel 4 nicht zentralsymmetrisch auf dem Verschlussabschnitt 2 angeordnet, sonder exzentrisch. Die einzelnen Merkmale der beschriebenen Ausführungsformen des Verschlussabschnitts 2 können je nach Bedarf miteinander kombiniert werden. Auch können verschiedene Ausführungsformen eines Verschlussabschnitts 2 auf demselben Implantat vorhanden sein.

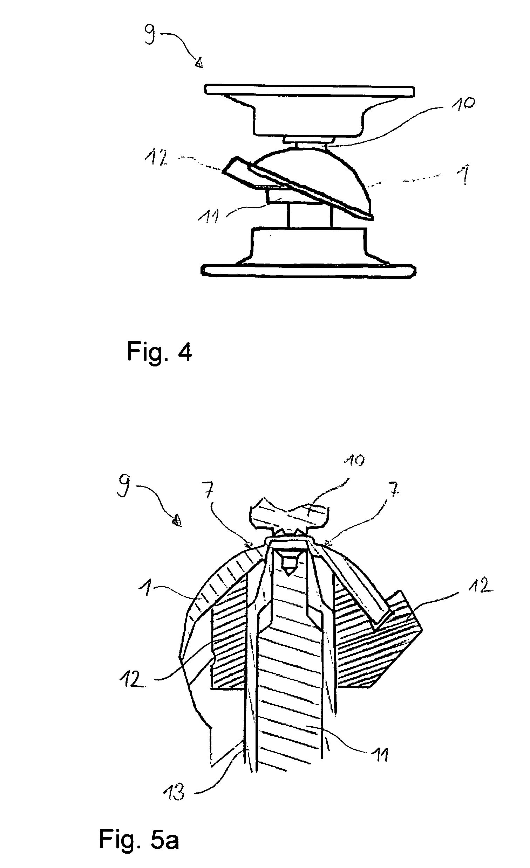

Figur 4 zeigt eine Vorrichtung 9 zur Herstellung eines Implantats 1. Das Implantat 1 ist dabei zwischen den beiden Prägestempeln 10, 11 eingespannt. Stützelement 12 stützt dabei das Implantat 1 zusätzlich ab.

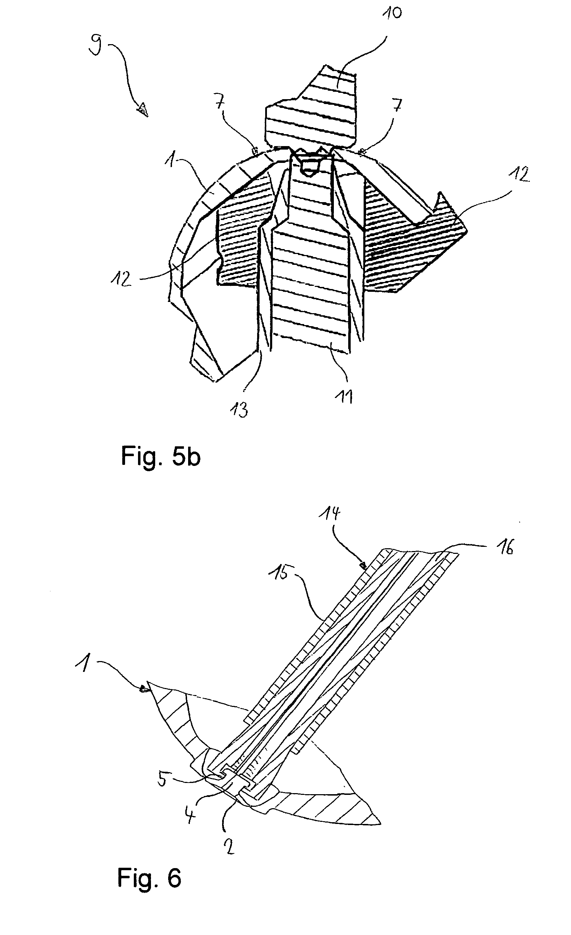

Figur 5 zeigt ein Ausführungsbeispiel des erfindungsgemässen Verfahrens anhand eines Schnittbilds durch eine Vorrichtung. In Figur 5a ist die Situation vor der Materialumformung zu sehen. Das durch ein erstes Fertigungsverfahren hergestellte Halbfabrikat des Implantats 1 wird in die Vorrichtung 9 zwischen den Prägestempeln 10,11 eingespannt. Dabei wird das Implantat 1 durch das Stützelement 12 zusätzlich abgestützt. Durch das Führungselement 13 erfolgt die korrekte Ausrichtung des Implantats 1 zu den Stempeln 10,11. Figur 5b zeigt die Vorrichtung 9 und das Implantat 1 während der Materialumformung. Dabei werden die beiden Prägestempel 10,11 gegeneinander gedrückt. Die Stützelemente 12,13 stützen dabei der Sollbruchlinie umgebende Wandabschnitte 7.

In Figur 6 ist ein Instrument 14 im Schnittbild gezeigt, welches mit einem Verschlussabschnitt 2 in wieder lösbarer Verbindung steht. Das Instrument 14 umfasst einen länglichen Griff 15 sowie Eingriffmittel 16. Bei der gezeigten Ausführungsform des Instruments 14 besteht das Eingriffmittel 16 aus einem länglichen Hohlkörper mit wenigstens dem gleichen Querschnitt wie die Angriffsmittel 4. Der Griff 15 ist zumindest im Bereich von Eingriffmittel 16 ebenfalls als Hohlkörper ausgebildet, der über dem Eingriffmittel 16 verschiebbar angeordnet ist. Beim dargestellten Beispiel ist der Griff 15 in diesem Bereich in drei Teile geteilt, welche durch sich verjüngende Schlitze voneinander getrennt sind. Durch Überschieben des Eingriffmittels 16 durch den Griff 15 verengt sich dessen Querschnitt beziehungsweise werden die drei Teile konzentrisch gegeneinander gedrückt. Dadurch kann das Eingriffmittel 16 an das Angriffsmittel 4 eingreifen. Bevorzugt weist das Eingriffmittel 16 zusätzliche Mittel auf, die in den Hinterschnitt 5 des Angriffsmittels 4 eingreifen können.

## Patentansprüche

1. Verfahren zum Herstellen eines Implantats (1) mit wenigstens einem Wandabschnitt, der wenigstens eine Sollbruchlinie (3) aufweist, welche zum Freilegen einer Öffnung oder Aussparung einen unter Krafteinwirkung aus dem Wandabschnitt entfernbaren Verschlussabschnitt (2) abgrenzt, **dadurch gekennzeichnet, dass**
- zuerst in einem vorzugsweise spanabhebenden Fertigungsverfahren ein Halbfabrikat des Implantats (1) hergestellt wird, und
- dass anschliessend die Sollbruchlinie (3) erzeugt wird, indem durch Materialumformung im Bereich der Sollbruchlinie (3) eine Reduzierung der Wandstärke im Wandabschnitt erfolgt.

2. Verfahren nach Anspruch 1, **dadurch gekennzeichnet, dass** die Materialumformung durch Druckformung im kalten oder warmen Zustand erfolgt, insbesondere durch einen Pressvorgang.

3. Verfahren nach einem der Ansprüche 1 oder 2, **dadurch gekennzeichnet, dass** die Materialumformung derart erfolgt, dass die Umformkräfte beziehungsweise der Materialfluss gezielt in den Verschlussabschnitt (2) oder das Implantat (1) geleitet werden.

4. Verfahren nach einem der Ansprüche 1 bis 3, **dadurch gekennzeichnet, dass** der entfernbare Verschlussabschnitt (2) derart hergestellt wird, dass wenigstens ein Angriffsmittel (4) zum Aufsetzen eines Instruments, vorzugsweise ein Materialvorsprung mit Hinterschnitt, gebildet wird.

5. Verfahren nach einem der Ansprüche 1 bis 4, **dadurch gekennzeichnet, dass** die Wandstärke im Bereich der Sollbruchlinie (3) auf 0.01mm bis 1mm, vorzugsweise 0.1mm bis 0.3mm reduziert wird.

6. Verfahren nach einem der Ansprüche 1 bis 5, **dadurch gekennzeichnet, dass** der Verschlussabschnitt (2) bei der Materialumformung zusätzlich konvex, konkav, wellenartig oder gerade gebogen wird.

7. Verfahren nach einem der Ansprüche 1 bis 6, **dadurch gekennzeichnet, dass** der die Sollbruchlinie (3) umgebende Wandabschnitt bei der Materialumformung abgestützt wird.

8. Implantat mit wenigstens einem Wandabschnitt, der wenigstens eine Sollbruchlinie aufweist, welche zum Freilegen einer Öffnung oder Aussparung einen unter Krafteinwirkung aus dem Wandabschnitt entfernbaren Verschlussabschnitt abgrenzt, **dadurch gekennzeichnet, dass** die Sollbruchlinie durch Materialumformung hergestellt ist.

9. Implantat nach Anspruch 8, **dadurch gekennzeichnet, dass** das Implantat eine Gelenkpfanne, bevorzugt eine Hüftgelenkpfanne ist.

10. Implantat nach einem der Ansprüche 8 oder 9, **dadurch gekennzeichnet, dass** der durch die Sollbruchlinie abgegrenzte
Verschlussabschnitt kreisförmig, oval, elliptisch, polygonal oder als Kombination davon ausgebildet ist.

11. Implantat nach einem der Ansprüche 8 bis 10, **dadurch gekennzeichnet, dass** der durch die Sollbruchlinie abgegrenzte Verschlussabschnitt mindestens teilweise durch eine Aussenkante des Implantats begrenzt ist.

12. Implantat nach einem der Ansprüche 8 bis 11, **dadurch gekennzeichnet, dass** das Implantat (1) aus einem metallischen Werkstoff besteht, bevorzugt aus Titan, einer Titanlegierung, einer Cobalt-Chromlegierung, einer Magnesiumlegierung und/oder einer Stahllegierung.

13. Implantat nach einem der Ansprüche 8 bis 12, **dadurch gekennzeichnet, dass** das Implantat (1) aus einem nichtmetallischen Werkstoff besteht, bevorzugt Polyetheretherketon und/oder Polyoxymethylen.

14. Implantat nach einem der Ansprüche 8 bis 13, **dadurch gekennzeichnet, dass** der entfernbare Verschlussabschnitt (2) wenigstens ein Angriffsmittel zum Aufsetzen eines Instruments, bevorzugt einen Materialvorsprung mit Hinterschnitt aufweist.

## Claims

1. Method for producing an implant (1) comprising at least one wall portion which has a predetermined breaking line (3) that delimits a closure portion (2) that can be removed from the wall portion under the effect of force to expose an opening or clearance, **characterized in that**
- a semifinished implant (1) is first produced in a production process that preferably involves machining, and
- **in that** then the predetermined breaking line (3) is created by reducing the wall thickness in the wall portion by forming the material in the region of the predetermined breaking line (3).

2. Method according to Claim 1, **characterized in that** the forming of the material is performed by forming under pressure in the cold or hot state, in particular by a pressing operation.

3. Method according to either of Claims 1 and 2, **characterized in that** the forming of the material is performed by the forming forces or the material flow being specifically directed into the closure portion (2) or the implant (1).

4. Method according to one of Claims 1 to 3, **characterized in that** the removable closure portion (2) is produced in such a way that at least one engaging means (4) for placing on an instrument is formed, preferably a material projection with an undercut.

5. Method according to one of Claims 1 to 4, **characterized in that** the wall thickness in the region of the predetermined breaking line (3) is reduced to 0.01 mm to 1 mm, preferably 0.1 mm to 0.3 mm.

6. Method according to one of Claims 1 to 5, **characterized in that**, during the forming of the material, the closure portion (2) is additionally bent convexly, concavely, into a wavy form or into a straight form.

7. Method according to one of Claims 1 to 6, **characterized in that** the wall portion surrounding the predetermined breaking line (3) is supported during the forming of the material.

8. Implant comprising at least one wall portion which has at least one predetermined breaking line that delimits a closure portion that can be removed from the wall portion under the effect of force to expose an opening or clearance, **characterized in that** the predetermined breaking line is produced by forming the material.

9. Implant according to Claim 8, **characterized in that** the implant is a joint socket, preferably a hip joint socket.

10. Implant according to either of Claims 8 and 9, **characterized in that** the closure portion delimited by the predetermined breaking line is formed in a circular, oval, elliptical or polygonal shape, or as a combination thereof.

11. Implant according to one of Claims 8 to 10, **characterized in that** the closure portion delimited by the predetermined breaking line is bounded at least partially by an outer edge of the implant.

12. Implant according to one of Claims 8 to 11, **characterized in that** the implant (1) consists of a metallic material, preferably of titanium, a titanium alloy, a cobalt-chromium alloy, a magnesium alloy and/or a steel alloy.

13. Implant according to one of Claims 8 to 12, **characterized in that** the implant (1) consists of a nonmetallic material, preferably polyether ether ketone and/or polyoxymethylene.

14. Implant according to one of Claims 8 to 13, **characterized in that** the removable closure portion (2) has at least one engaging means for placing on an instrument, preferably a material projection with an undercut.

## Revendications

1. Procédé de fabrication d'un implant (1) qui présente au moins un segment de paroi dotée d'au moins une ligne (3) de rupture préférentielle qui délimite un segment de fermeture (2) qui peut être enlevé du segment de paroi sous l'action d'une force pour libérer une ouverture ou une découpe,
le procédé étant **caractérisé en ce que**
une ébauche d'implant (1) est d'abord fabriquée par un procédé de fabrication de préférence d'usinage par enlèvement de copeaux et
la ligne (3) de rupture préférentielle est ensuite formée en réduisant l'épaisseur du segment de paroi par déformation du matériau en zone de la ligne (3) de rupture préférentielle.

2. Procédé selon la revendication 1, **caractérisé en ce que** la déformation du matériau s'effectue par déformation sous pression à l'état froid ou chaud et en particulier par une opération de pressage.

3. Procédé selon l'une des revendications 1 ou 2, **caractérisé en ce que** la déformation du matériau s'effectue en orientant les forces de déformation ou l'écoulement du matériau de manière contrôlée dans le segment de fermeture (2) ou dans l'implant (1).

4. Procédé selon l'une des revendications 1 à 3, **caractérisé en ce que** le segment de fermeture (2) à enlever est réalisé en formant au moins un moyen d'engagement (4) destiné à l'application d'un instrument, et de préférence une saillie de matériau avec contre-dépouille.

5. Procédé selon l'une des revendications 1 à 4, **caractérisé en ce que** l'épaisseur de la paroi en zone de la ligne (3) de rupture préférentielle est réduite à 0,01 m - 1 mm et de préférence à 0,1 mm - 0,3 mm.

6. Procédé selon l'une des revendications 1 à 5, **caractérisé en ce que** lors de la déformation du matériau, le segment de fermeture (2) est de plus cintré de manière convexe, concave, en ondulations ou en ligne droite.

7. Procédé selon l'une des revendications 1 à 6, **caractérisé en ce que** le segment de paroi qui entoure la ligne (3) de rupture préférentielle est soutenu lors de la déformation du matériau.

8. Implant présentant au moins un segment de paroi dotée d'au moins une ligne de rupture préférentielle qui délimite un segment de fermeture qui peut être enlevé du segment de paroi sous l'action d'une force pour libérer une ouverture ou une découpe,
**caractérisé en ce que**
la ligne de rupture préférentielle est réalisée par déformation du matériau.

9. Implant selon la revendication 8, **caractérisé en ce que** l'implant est une cuvette d'articulation et de préférence une cuvette d'articulation de la hanche.

10. Implant selon l'une des revendications 8 ou 9, **caractérisé en ce que** le segment de fermeture délimité par la ligne de rupture préférentielle a une forme circulaire, ovale, elliptique, polygonale ou une combinaison de ces formes.

11. Implant selon l'une des revendications 8 à 10, **caractérisé en ce que** le segment de fermeture délimité par la ligne de rupture préférentielle est délimité au moins en partie par une arête extérieure de l'implant.

12. Implant selon l'une des revendications 8 à 11, **caractérisé en ce que** l'implant (1) est constitué d'un matériau métallique, de préférence de titane, d'un alliage de titane, d'un alliage de cobalt et de chrome, d'un alliage de magnésium et/ou d'un alliage d'acier.

13. Implant selon l'une des revendications 8 à 12, **caractérisé en ce que** l'implant (1) est constitué d'un matériau non métallique, de préférence de polyétheréthercétone ou de polyoxyméthylène.

14. Implant selon l'une des revendications 8 à 13, **caractérisé en ce que** le segment de fermeture (2) apte à être retiré présente au moins un moyen d'engagement qui permet d'appliquer un instrument, de préférence une saillie de matériau doté d'une contre-dépouille.
